(19) Europäisches Patentamt
European Patent Office
Office européen des brevets

(11) **EP 3 366 196 A1**

(12) **DEMANDE DE BREVET EUROPEEN**

(43) Date de publication:
**29.08.2018 Bulletin 2018/35**

(51) Int Cl.:
*A61B 5/00* (2006.01)       *G01N 21/3577* (2014.01)

(21) Numéro de dépôt: **17157500.4**

(22) Date de dépôt: **22.02.2017**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**MA MD**

(71) Demandeur: **Medical Scientific Innovations - MSI 75016 Paris (FR)**

(72) Inventeurs:
• **AHMED, Pasem**
  **92230 GENNEVILLIERS (FR)**
• **CHASTANIER, Pierre**
  **75008 PARIS (FR)**
• **BAILLIART, Olivier**
  **75016 PARIS (FR)**
• **POUGET, Raymond**
  **92100 BOULOGNE-BILLANCOURT (FR)**
• **DIXMIER, Michel**
  **75018 PARIS (FR)**
• **BRAHIMI, Mohand Boujema**
  **75018 PARIS (FR)**

(74) Mandataire: **Gauchet, Fabien Roland et al Brandon IP 64, rue Tiquetonne 75002 Paris (FR)**

(54) **PROCEDE ET DISPOSITIF D'INTRODUCTION D'AU MOINS UNE SUBSTANCE EXOGENE DOSEE, DANS UN LIQUIDE ENDOGENE**

(57) La présente invention concerne un dispositif de reconnaissance, de mesure et de dosage d'au moins une substance exogène dans un liquide endogène. Selon l'invention le dispositif comprend : - un moyen d'aspiration (1) d'un liquide endogène contenant au moins une substance exogène - un moyen de collecte (2) du liquide endogène contenant au moins une substance exogène ; - un spectromètre (3) de préférence Raman ou infrarouge apte à acquérir et analyser ledit liquide collecté ; - un moyen de mémorisation de données de type base de données comprenant des spectres relatifs à au moins une substance exogène ; - un moyen de comparaison entre le ou les spectres acquis et le ou les spectres de la base de données ; - un moyen (4) d'introduction contrôlée d'au moins une substance exogène dans le liquide endogène, en fonction du résultat de ladite comparaison.

L'invention vise en outre le procédé mis en oeuvre par un tel dispositif.

FIG. 1

## Description

## DOMAINE TECHNIQUE DE L'INVENTION

L'invention se rapporte au domaine du dosage d'une substance exogène dans un liquide endogène. Plus précisément l'invention vise la mesure et l'introduction contrôlée d'une substance exogène telle qu'un antibiotique dans un liquide endogène tel que le sang. Une introduction contrôlée, ou dosage, quasi instantanée est visée par l'invention.

## ETAT DE LA TECHNIQUE ANTERIEURE

De façon connue, l'administration de substances exogènes telles que des antibiotiques dans le sang se fait par perfusion intermittente ou bien par perfusion continue. Il est tout à fait nécessaire de contrôler les concentrations dites sériques des dits antibiotiques.

Actuellement ces dosages se font par des appareillages basés sur la spectrométrie de masse, avec des méthodes chères donc non réalisées fréquemment. On prend un échantillon de sang que l'on analyse par exemple avec un spectromètre Raman puis en fonction du résultat on injecte une dose d'antibiotique déterminée au patient. Cette opération est réalisée par exemple toutes les quatre heures.

Dans le cas de chocs septiques, il est nécessaire d'administrer le ou les antibiotiques dans l'heure qui suit le choc ; ceci n'est malheureusement pas toujours possible ni simple.

Afin de caractériser et de doser des échantillons, on peut utiliser la spectroscopie Raman ou encore la spectroscopie infrarouge. Concernant la spectroscopie à infrarouge l'un des problèmes récurrent réside dans le choix de la longueur d'onde ; en effet les bandes d'absorption sont larges et se chevauchent. On peut identifier des composés purs ou des impuretés ; pour la plupart des composés organiques ; le spectre comporte plusieurs bandes correspondant à plusieurs critères qui peuvent donc être utilisés dès lors qu'il n'existe pas de chevauchement avec les pics d'absorption d'autres substances présentes dans l'échantillon.

Il est connu d'utiliser la spectroscopie infrarouge pour des analyses à la fois qualitatives et quantitatives dans les domaines de la pharmacie, de l'agriculture, des industries alimentaires. Dans l'industrie pharmaceutique il est fréquent d'utiliser des méthodes fondées sur la spectroscopie infrarouge pour doser les principes actifs des comprimés ; dans l'agriculture on l'emploie par exemple pour déterminer la teneur en protéine brute contenue dans l'ensilage de foin sec. Dans l'industrie alimentaire on s'en sert pour le dosage des graisses, des protéines et du lactose dans le lait ou encore pour suivre, de façon non-destructrice, les teneurs d'éthanol dans les processus de fermentation. Cette méthode d'investigation présente l'avantage d'être non-destructive, non invasive. Mais, outre les inconvénients déjà mentionnés, cette méthode requiert un ensemble d'échantillons pour l'étalonnage, de préférence obtenus à partir d'échantillons réels dans lesquels les constituants à analyser sont en concentrations suffisamment différentes.

La spectroscopie Raman dont il ne sera ici pas rappelé le principe physique, est utilisée depuis la fin des années 1980. Il est intéressant de noter que cette méthode est, comme la spectrométrie infrarouge, non destructive et non invasive. Elle est en outre de mise en oeuvre aisée, ne requiert qu'une faible quantité d'échantillon, de l'ordre du microgramme. Elle peut être utilisée dans un grand nombre de cas : matériaux hétérogènes, échantillons dont la structure peut être orientée ou non. Elle peut être couplée avec d'autres méthodes analytiques, et offre la possibilité de mesures in situ. Elle est applicable aux solides atomiques et est utilisable sur des échantillons de très petite taille, jusqu'à $10^{-18}m^3$. Cette technique permet en outre de travailler en milieu hostile, notamment à haute température en présence de phénomènes radioactifs ou sous atmosphère contrôlée. Par ailleurs cette méthode est sensible aux petites structures : identification des systèmes amorphes, analyses des films très fins pour lesquels les méthodes de diffraction sont parfois difficiles à réaliser.

Ainsi la possibilité d'analyser rapidement une large gamme d'échantillons et la capacité de collecter un grand nombre de spectres avec une haute résolution en une seule mesure ont rendu la technique Raman accessible à un nombre beaucoup plus étendu de domaines scientifiques.

On connait des appareils portables faisant appel à la spectroscopie Raman qui sont souvent utilisés pour l'identification des matériaux cosmétiques, pharmaceutiques et chimiques. Ces dispositifs permettent notamment l'identification rapide et précise des matières premières, l'inspection des produits finis et la détection de contrefaçons. Ces analyseurs Raman portables permettent aussi de réaliser des contrôles qualité, directement sur site et en quelques secondes, sur une grande variété de solides, liquides, poudres et autres substances. Il est d'ailleurs possible de mesurer à travers des emballages transparents, tels que des sacs en plastique et des bouteilles en verre.

De façon connue la spectroscopie infrarouge et la spectroscopie Raman permettent des analyses en temps réel avec des appareils portables, ce qui est très intéressant. A titre de comparaison, la spectroscopie Raman permet d'obtenir à peu près le même type de résultats que la spectroscopie infrarouge, bien que le principe de la méthode soit différent. Moins utilisée en raison de son coût, la spectroscopie Raman présente toutefois des avantages par rapport à la spectroscopie infrarouge.

[0011] - Emploi de l'eau, excellent solvant pour la spectroscopie Raman. En effet la présence d'eau n'est pas gênante car l'eau diffuse très peu en Raman ;

Utilisation de cuves de verre : le verre est transparent dans les domaines spectraux concernés et son spectre Raman est très faible.

les échantillons peuvent être utilisés sous n'importe quelle forme, sans être dilués ni altérés.

Les spectres sont généralement plus simples, de sorte que les chevauchements de bandes sont beaucoup moins fréquents.

Possibilité d'étudier des modes de vibration totalement symétriques, invisibles en infrarouge.

Obtention de renseignements complémentaires par polarisation de la lumière.

L'effet Raman est indépendant de la longueur d'onde excitatrice utilisée, ce qui permet de supprimer certains phénomènes indésirables (fluorescence, décomposition des substances colorées...) en choisissant une longueur d'onde adéquate.

Aucune polarisation permanente des molécules n'est nécessaire.

Utilisation d'un seul appareil et d'un seul balayage en nombre d'onde pour couvrir la totalité du spectre des fréquences de vibration, ceci en raison de la nature même de l'effet Raman.

Les intensités sont directement proportionnelles à la concentration, contrairement à la spectroscopie infrarouge, pour laquelle on doit appliquer la loi de Beer. Ainsi l'analyse quantitative par spectroscopie Raman est souvent plus aisée et plus précise.

Ces avantages sont toutefois contrebalancés par la gêne provoquée par quelques phénomènes tels que:

- L'émission du corps noir (par échauffement de l'échantillon) ;

- La fluorescence qui est beaucoup plus intense que l'effet Raman lorsqu'elle se produit mais qui peut être évitée en changeant de longueur d'onde ;

- Les réactions photochimiques (décomposition des substances colorées...) ;

Les réactions multiphotoniques ;
La décomposition des échantillons par échauffement.

Cependant les progrès technologiques tendent à réduire le surcoût de la spectroscopie Raman, et accroissent ses performances. A titre d'exemple, le spectromètre Raman à Transformée de Fourier présente des avantages supplémentaires connus suivants :

- Le problème de fluorescence ne se pose plus grâce à l'utilisation d'une raie excitatrice peu énergique. En effet en spectroscopie Raman à transformée de Fourier, on se sert de radiations excitatrices dans le proche infrarouge. Ceci permet d'éviter les problèmes liés à la fluorescence de l'échantillon pouvant intervenir lorsqu'on utilise des lasers fonctionnant dans la région des radiations visibles.

- Les substances colorées ne risquent pas de se décomposer, pour la raison évoquée ci-dessus ;

- L'analyse est plus rapide car tous les éléments spectraux sont mesurés simultanément ;

- La sensibilité est meilleure car une plus grande quantité de photons est détectée simultanément ;

- les fréquences sont détectées avec précision grâce à un laser qui mesure la position du miroir mobile de l'interféromètre.

Dans un article intitulé in vivo blood glucose quantification using Raman spectroscopie' ' quantification in vivo de glucose dans le sang par utilisation de la spectroscopie Raman', publié en octobre dans la revue PLOS ONE volume 7 issue 10 e48127, Jingwei Shao et son équipe ont proposé une méthode de spectroscopie Raman qui permet la détection in vivo de la concentration plasmatique de glucose. Cette méthode est intéressante pour les raisons évoquées ci-avant mais cependant elle est limitée à la concentration plasmatique de glucose.

On ne connait pas de méthode ni d'appareil qui permette une détection in vivo de substance exogène telle que par exemple au moins un antibiotique.

On connait le brevet US 7 972 296 B2 qui décrit une méthode et un système pour le dosage et l'introduction contrôlée de glucose en cas de glycémie, basés sur une spectrophotométrie ou une spectrométrie dans le moyen infra rouge. On injecte ici du glucose ou de l'insuline grâce à des poches ou à un pousse-seringue. Des moyens aptes à mesurer au moins un paramètre physiologique, ou un analyte sont utilisés. On administre ainsi une substance endogène (glucose ou équivalent) dans un liquide endogène (sang du patient).

## EXPOSE DE L'INVENTION

L'invention vise à remédier aux inconvénients de l'état de la technique et notamment à proposer un dispositif apte à la détection, la mesure et le dosage d'au moins une substance exogène dans un liquide endogène.

Pour ce faire est proposé selon un premier aspect de l'invention un dispositif (portatif) de reconnaissance, de mesure et de dosage d'au moins une substance exogène dans un liquide endogène qui comprend :
- un moyen d'aspiration d'un liquide endogène contenant au moins une substance exogène - un moyen de collecte dudit liquide endogène ; - un spectromètre Raman ou infrarouge apte à acquérir et analyser ledit liquide collecté ; - un moyen de mémorisation de données de type base de données comprenant des spectres relatifs à au moins une substance exogène ; - un moyen de comparaison entre le ou les spectres acquis et le ou les spectres de la base de données ; - au moins un moyen d'introduction contrôlée d'au moins une substance exogène dans le liquide endogène, en fonction du résultat de ladite comparaison spectrale.

De façon préférée, ledit spectromètre est du type Raman ou infrarouge.

A titre illustratif, le liquide exogène comprend au moins un antibiotique tandis que le liquide endogène est constitué du sang d'un patient. Sans sortir du cadre de l'invention, le liquide exogène peut comprendre les chimiothérapies. D'autres composés peuvent bien entendu être contrôlés et monitorés (au sens médical courant) en temps réel ; diverses molécules présentes dans des fluides corporels peuvent être identifiées et quantifiées en temps réel, par exemple des cellules du système immunitaire.

Le dispositif selon l'invention peut comprendre en outre au moins un moyen d'affichage de la mesure et/ou du dosage et/ou de la nature d'au moins une substance exogène contenue dans le liquide endogène. Tout écran tactile ou non peut constituer le moyen d'affichage.

Selon un mode préféré de réalisation de l'invention, le ou les moyen(s) d'introduction contrôlée permet une vitesse d'introduction variable et contrôlée de la substance exogène.

Avantageusement, le ou les moyen(s) d'introduction contrôlée peut être asservi au spectromètre Raman ou infrarouge.

De façon intéressante, le dispositif selon l'invention peut comprendre en outre un dispositif apte à faire varier la température de la substance exogène dans son milieu, afin de stabiliser ses propriétés physiques.

Selon une autre caractéristique de l'invention, ledit au moins un moyen d'introduction contrôlée permet un contrôle dans le temps et/ou en quantité de la substance exogène.

Par ailleurs, lesdits spectres Raman ou infrarouge sont corrélés aux variations de température et/ou aux variations d'acidité du liquide endogène.

En outre, le dispositif selon l'invention peut comprendre des moyens de centrifugation du liquide endogène.

Selon un mode particulier de réalisation de l'invention, le dispositif comprend plusieurs moyens d'introduction contrôlée d'une substance exogène, montés sur une structure porteuse, et chacun connecté au moyen de comparaison et de calcul.

L'invention vise par ailleurs un procédé de modification de la composition d'un liquide endogène par une substance exogène qui comprend notamment les étapes suivantes : - collecte du liquide endogène à l'entrée d'un dispositif tel que décrit ; analyse dudit liquide par spectrométrie, de préférence Raman ou infrarouge ; - mémorisation de spectres dans une base de données, lesdits spectres étant relatifs à au moins une substance exogène ; - comparaison d'au moins un spectre mémorisé avec au moins un spectre de référence d'un élément du liquide endogène ; - introduction contrôlée en vitesse d'au moins une substance exogène dans le liquide endogène qui sort du dispositif, en fonction du résultat de ladite comparaison spectrale.

**BREVE DESCRIPTION DES FIGURES**

[001]D'autres caractéristiques, détails et avantages de l'invention ressortiront à la lecture de la description qui suit, en référence aux figures annexées, qui illustrent :

- la figure 1, une vue extérieure du dispositif selon un mode de réalisation de l'invention ;
- la figure 2, un schéma du principe de fonctionnement d'un spectromètre Raman ;
- la figure 3, des spectres Raman de trois substances exogènes de type antibiotiques;
- la figure 4, un exemple de spectre Raman obtenu pour deux substances exogènes différentes ; et
- la figure 5, une vue en perspective d'un mode particulier de réalisation de l'invention.

Pour plus de clarté, les éléments identiques ou similaires sont repérés par des signes de référence identiques sur l'ensemble des figures.

**DESCRIPTION DETAILLEE D'UN MODE DE REALISATION**

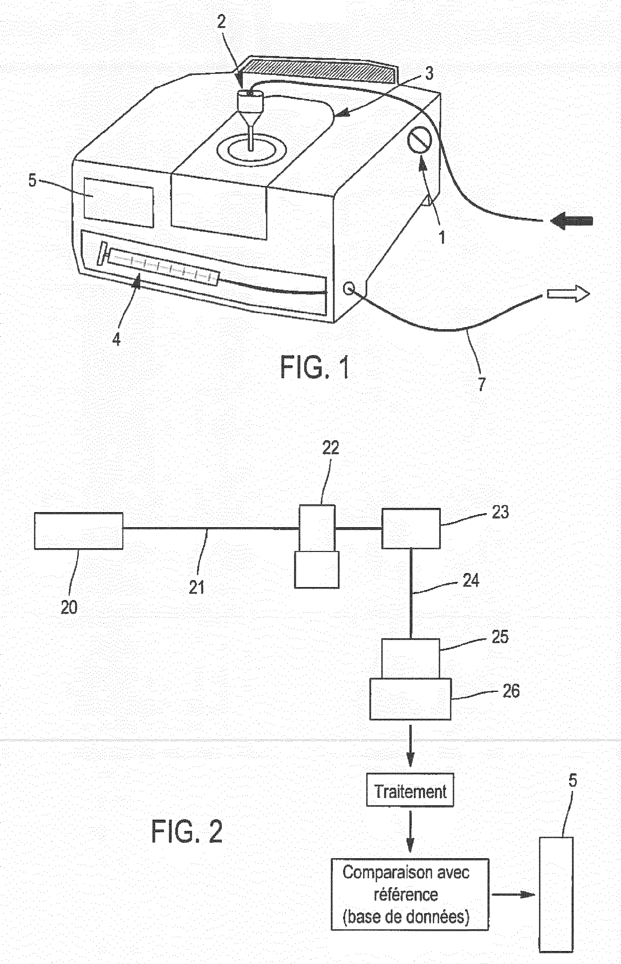

La figure 1 illustre un dispositif conforme à l'invention ; plus précisément le dispositif comprend un moyen d'aspiration 1 d'un fluide endogène tel que le sang. Ce moyen d'aspiration 1 est connecté à un collecteur 2 qui permet de collecter une quantité déterminée (ou échantillon) du fluide endogène. L'échantillon recueilli est analysé par un spectromètre Raman 3 disposé préférentiellement dans le dispositif 1 afin de constituer un ensemble autonome dédié. Bien entendu toutes les connections et autres liaisons appropriées sont prévues afin que le fluide collecté puisse être analysé par le spectromètre Raman. Ces connections et liaisons ne seront pas décrites en détail car à la portée de l'homme de métier.

Le dispositif comprend en outre un moyen d'introduction contrôlée du fluide exogène soit directement dans le fluide endogène alors qu'il circule dans le dispositif lui-même, soit dans un circuit distinct. Dans tous les cas il s'agit de doser et donc de contrôler le fluide exogène qui doit être introduit dans le fluide endogène ou dans le corps d'un patient. Pour ce faire, un moyen de type pousse seringue 4 est utilisé. La méthode utilisée sera décrite ci-après. Avantageusement le fonctionnement du pousse-seringue est asservi au spectromètre Raman, comme il sera explicité ci-après.

Par ailleurs le dispositif selon l'invention est préférentiellement pourvu d'un moyen d'affichage 5 de la mesure et/ou de la quantité et/ou du dosage et/ou de la nature d'au moins une substance exogène présente dans le fluide endogène. Le moyen d'affichage peut être un écran tactile ou non.

De préférence et pour les avantages évoqués ci-dessus, un spectromètre Raman est utilisé.

La figure 2 illustre le principe de fonctionnement du spectromètre Raman dans le cadre de l'invention. Etant donné que dans une application préférée de l'invention, c'est le plasma sanguin qui est visé en tant que liquide endogène, il apparaît que le spectromètre Raman est dans ce cas d'application le moyen le plus adapté pour la mise en oeuvre de l'invention. On sait en effet que l'eau constitue plus de 90% du plasma sanguin, d'où le choix du spectromètre Raman. Sans sortir du cadre de l'invention, un spectromètre infrarouge peut être utilisé.

Selon la figure 2, Les radiations d'une source Laser 20 sont véhiculées à travers une fibre optique 21 qui traverse l'échantillon recueilli 22, à analyser. Il se crée alors un échauffement dans l'échantillon, la lumière produite est captée par un capteur 23 et acheminée par une fibre optique 24 jusqu'à un séparateur 25 couplé à un détecteur 26 qui fournit des données relatives à l'échantillon recueilli 22. Ces données sont alors traitées par un système informatique approprié, non représenté structurellement. Les données consistent en des spectres.

Le traitement des données consiste en une comparaison entre les spectres relatifs aux données relatives à l'échantillon recueilli avec des spectres issus d'une base de données, stockés dans le système informatique. Le résultat de cette comparaison peut présenter différentes formes, qui peuvent être affichées sur le moyen 5.

Il s'agit ici de configurer un spectromètre Raman afin de déterminer en temps quasi réel le dosage, la concentration de la substance exogène dans le liquide endogène en sortie du dispositif selon l'invention.

Comme déjà rapidement évoqué, le fonctionnement du pousse-seringue 4 dépend notamment du résultat de la comparaison entre des spectres connus et des spectres du liquide collecté. Un pousse-seringue électrique peut ainsi être choisi pour cette fonctionnalité. Plus précisément le pousse seringue 4 peut avantageusement réaliser une modification de la vitesse de perfusion de la substance exogène en fonction de la concentration sanguine mesurée par le dispositif selon l'invention. Ainsi, quels que soient les autres paramètres, le taux de substance exogène administré est constant.

Une adaptation de la vitesse de perfusion est opérée selon des objectifs préétablis et sélectionnés. Dans le cas d'application d'une antibiothérapie, la prescription constitue la base des objectifs préétablis.

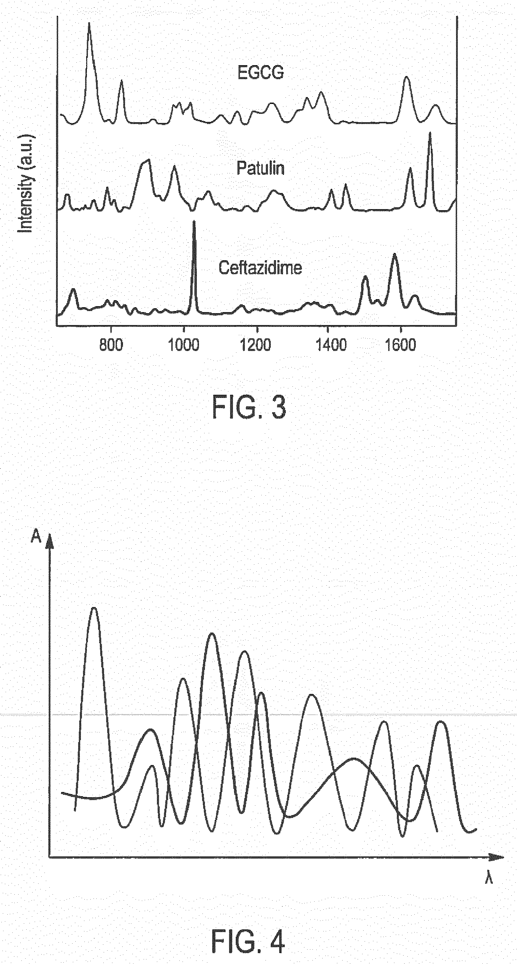

Comme montré sur la figure 3, chaque antibiotique présente un spectre spécifique c'est-à-dire une signature ou empreinte caractéristique. De façon connue la figure 3 donne des courbes de l'intensité en fonction du nombre d'onde (en cm$^{-1}$). La figure 3 donne trois spectres définis par l'intensité en fonction du décalage Raman (nombre d'intensité) ; les trois antibiotiques illustrés correspondent respectivement au EGCG (courbe supérieure), au Patulin (courbe intermédiaire) et au Ceftazidime (courbe inférieure).

Il est donc nécessaire de constituer une base de données constituée de spectres Raman de différentes substances exogènes susceptibles d'être recueillies pour analyse, dans le cadre de l'invention.

Concernant l'élaboration d'une base de données, il est ici illustré le cas de l'élaboration d'une base de données dans le domaine des antibiotiques. En effet en antibiothérapie il est crucial d'évaluer les concentrations thérapeutiques propres à chaque antibiotique ; l'objectif étant de connaître l'intervalle des concentrations nécessaires pour la courbe de calibration respective à chaque antibiotique.

De façon avantageuse dans le cas de la mesure et du dosage d'antibiotiques dans le sang, la base de données prend en compte les variations de température et d'acidité du sang afin d'interpréter les concentrations plasmatiques en antibiotiques.

Chaque antibiotique (à partir d'une solution pure) sera analysé par spectroscopie Raman, pour un intervalle de concentrations préalablement définies. Pour tenir compte des variations incontrôlées inhérentes à l'expérimentation, il est nécessaire d'accumuler les spectres de nombreux échantillons. A des fins de fiabilité, ces analyses devront donc être répétées plusieurs fois, dans les mêmes conditions. La figure 4 illustre les résultats d'une telle accumulation de spectres. La température représente un autre paramètre à inclure. En effet, il est essentiel que l'analyse se fasse à différentes températures (de 34°C +/- 0,1 à 41°C). Ceci permettrait par la suite d'assurer la fiabilité des données, en prenant en compte la température corporelle du patient.

Par ailleurs, une étape d'étalonnage des données est indispensable avant toute utilisation en routine. L'utilisation d'une courbe d'étalonnage permet de résoudre tous les problèmes liés à la non-linéarité de la courbe d'absorbance ou aux concentrations effectives des espèces absorbantes ; cela signifie que toute mesure pour une solution de titre inconnu, effectuée dans les mêmes conditions que pour une série d'étalons, permet de déduire directement d'après la courbe. La méthode préconisée nécessite que les mesures pour tous les étalons et tous les échantillons soient effectuées dans la même cellule, avec un même chemin optique, mais il n'est pas nécessaire de connaître ni les dimensions exactes de la cellule ni le coefficient d'absorption molaire pour la bande d'absorption choisie, car ce sont des constantes. La collection spectrale ainsi obtenue sera traitée par des méthodes chimiométriques. Cependant avant d'être exploitées, les données spectrales brutes feront l'objet de divers prétraitements dont les objectifs sont :

- Une atténuation ou une élimination de la non-linéarité présente entre les variables dépendantes et explicatives,
- Une élimination des interférences,
- Une élimination ou une atténuation du bruit aléatoire lié aux conditions expérimentales et au bruit électronique de l'appareil de mesure,

- Une réduction des corrélations entre les variables explicatives pour permettre l'application des techniques de régression linéaire multiple.

Les spectres collectés peuvent être représentés sous forme d'une matrice. Cette représentation matricielle permet ainsi de réaliser des analyses multivariées. Soit n échantillons analysés par un spectromètre Raman et p le nombre de longueurs d'onde différentes mesurées par l'instrument. La matrice spectrale nxp prend la forme suivante :

$$X = \begin{pmatrix} I_1^1 & \cdots & I_1^p \\ \vdots & \ddots & \vdots \\ I_n^1 & \cdots & I_n^p \end{pmatrix}$$

Chaque ligne correspond à un échantillon et chaque colonne représente les absorbances des échantillons pour une longueur d'onde donnée.

La stratégie la plus courante consiste à soumettre les données brutes à une ou plusieurs transformation(s) mathématique(s) destinée(s) à les rendre aptes à une modélisation linéaire. Le choix de la transformation est fonction du type de non-linéarité affectant les données et donc de la qualité des modèles prédictifs élaborés pour chaque prétraitement.

On met en oeuvre une méthode quantitative par analyses multivariées qui comprend une analyse de régression sur des échantillons de concentrations connues, et une prédiction d'échantillons inconnus à partir d'analyses d'échantillons de concentrations inconnues.

Il est ensuite indispensable de disposer de paramètres permettant de valider l'ajustement d'un modèle et/ou de comparer plusieurs modèles afin de choisir in fine celui qui propose les meilleurs résultats en termes de prédiction.

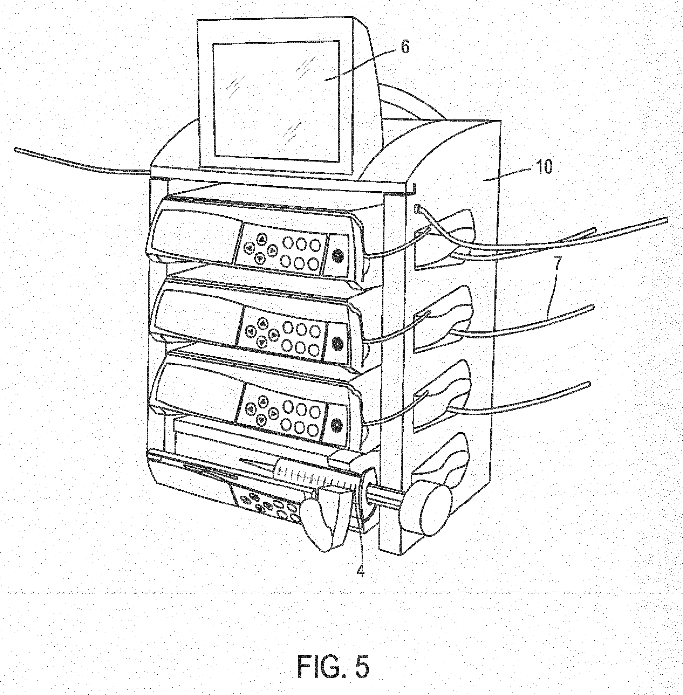

La figure 5 illustre un mode particulier de réalisation de l'invention qui comprend plusieurs moyens 4 d'introduction contrôlée d'une substance exogène dans le liquide endogène. Les moyens 4 sont ici des pousse-seringue, au nombre de quatre, montés sur une même structure porteuse 10. Le liquide exogène est introduit par un conduit 7 dans chaque moyen 4 associé. Il en ressort par un conduit non visible sur la figure 5, à une vitesse déterminée par le moyen de comparaison et de calcul. Un moyen d'affichage général 6 peut être prévu afin de visualiser les différents paramètres et résultats de calcul

Bien entendu le dispositif et le procédé selon l'invention ne se limitent pas aux cas d'application illustrés ci-avant ; l'invention permet toute analyse en temps réelle d'une substance exogène dans un liquide endogène associée à une introduction et/ou une injection asservie au résultat de l'analyse.

## Revendications

1. Dispositif de reconnaissance, de mesure et de dosage d'au moins une substance exogène dans un liquide endogène **caractérisé en ce qu'**il comprend :- un moyen d'aspiration (1) d'un liquide endogène contenant au moins une substance exogène ; - un moyen de collecte (2) dudit liquide endogène; - un spectromètre (3) apte à acquérir et analyser ledit liquide collecté ; - un moyen de mémorisation de données de type base de données comprenant des spectres relatifs à au moins une substance exogène ; - un moyen de comparaison entre le ou les spectres acquis et le ou les spectres de la base de données ; - au moins un moyen (4) d'introduction contrôlée d'au moins une substance exogène dans le liquide endogène, en fonction du résultat de ladite comparaison spectrale.

2. Dispositif selon la revendication 1 **caractérisé en ce que** ledit spectromètre est du type Raman ou infrarouge.

3. Dispositif selon la revendication 1 ou 2 **caractérisé en ce qu'**il comprend en outre un moyen d'affichage (5,6) de la mesure et/ou du dosage et/ou de la nature d'au moins une substance exogène contenue dans le liquide endogène.

4. Dispositif selon l'une des revendications précédentes **caractérisé en ce que** ledit au moins un moyen (4) d'introduction contrôlée permet une vitesse d'introduction variable et contrôlée de la substance exogène.

5. Dispositif selon l'une des revendications précédentes **caractérisé en ce que** ledit au moins un moyen (4) d'introduction contrôlée est asservi au spectromètre.

6. Dispositif selon l'une quelconque des revendications précédentes **caractérisé en ce qu'**il comprend en outre un dispositif apte à faire varier la température de la substance exogène dans son milieu, afin de stabiliser ses propriétés physiques.

7. Dispositif selon l'une des revendications précédentes **caractérisé en ce que** ledit au moins un moyen (4) d'introduction contrôlée permet un contrôle dans le temps et/ou en quantité de la substance exogène.

8. Dispositif selon l'une des revendications précédentes **caractérisé en ce que** lesdits spectres sont corrélés aux variations de température et/ou aux variations d'acidité du liquide endogène.

9. Dispositif selon l'une quelconque des revendications précédentes **caractérisé en ce qu'**il comprend en outre des moyens de centrifugation du liquide endogène.

**10.** Dispositif selon l'une quelconque des revendications précédentes **caractérisé en ce qu'**il comprend plusieurs moyens (4) d'introduction contrôlée d'une substance exogène, montés sur une structure porteuse (10), et chacun connecté au moyen de comparaison et de calcul.

**11.** Dispositif selon l'une quelconque des revendications précédentes **caractérisé en ce que** le liquide exogène comprend au moins un antibiotique.

**12.** Procédé de modification de la composition d'un liquide endogène par une substance exogène **caractérisé en ce qu'**il comprend les étapes : - aspiration du liquide endogène à l'entrée d'un dispositif selon l'une quelconque des revendications 1 à 11 ; - collecte dudit liquide ; - analyse dudit liquide par spectrométrie de préférence Raman ou infrarouge ; - mémorisation de spectres dans une base de données, lesdits spectres étant relatifs à au moins une substance exogène ; - comparaison d'au moins un spectre mémorisé avec au moins un spectre de référence d'un élément du liquide endogène ; - introduction contrôlée en vitesse d'au moins une substance exogène dans le liquide endogène qui sort du dispositif selon l'une quelconque des revendications 1 à 11, en fonction du résultat de ladite comparaison spectrale.

FIG. 1

FIG. 2

Traitement

Comparaison avec référence (base de données)

FIG. 3

FIG. 4

FIG. 5

Europäisches
Patentamt

European
Patent Office

Office européen
des brevets

# RAPPORT DE RECHERCHE EUROPEENNE

Numéro de la demande

EP 17 15 7500

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2012/203089 A1 (RULE PETER [US] ET AL) 9 août 2012 (2012-08-09) | 1-9,12 | INV. A61B5/00 G01N21/3577 |
| Y | * alinéas [0042], [0048] - [0049], [0050], [0059], [0070] - [0075], [0094], [0138] - [0160], [0173], [0238] - [0249]; figures 1,24-27 * <br> * alinéa [0240] * | 10,11 | |
| X | WO 2007/052255 A2 (FENSTER MARK [IL]; KABIRI ILAN [IL]) 10 mai 2007 (2007-05-10) | 1,12 | |
| Y | * alinéas [0030] - [0032], [0038], [0041] - [0043]; figures 1,5 * | 10,11 | |
| Y | WO 2005/025413 A2 (HOLMES ELIZABETH A [US]) 24 mars 2005 (2005-03-24) <br> * page 34, ligne 24 - page 35, ligne 2 * | 11 | |
| Y | ZALESSKAYA G A ET AL: "Determination of the concentration of cephalosporin antibiotics in blood by infrared spectroscopy", JOURNAL OF APPLIED SPECTROSCOPY, KLUWER ACADEMIC PUBLISHERS-PLENUM PUBLISHERS, NE, vol. 74, no. 4, 1 juillet 2007 (2007-07-01), pages 567-570, XP019574524, ISSN: 1573-8647 <br> * abrégé * | 11 | DOMAINES TECHNIQUES RECHERCHES (IPC) <br><br> A61B <br> G01N |
| Y | WO 2005/102441 A1 (UNIV TEXAS [US]; DENT DANIEL L [US]; ALMEDA JOSE LUIS [US]) 3 novembre 2005 (2005-11-03) <br> * page 10, lignes 34-35 * | 11 | |

Le présent rapport a été établi pour toutes les revendications

| Lieu de la recherche | Date d'achèvement de la recherche | Examinateur |
|---|---|---|
| Munich | 27 juillet 2017 | Meacher, David |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 17 15 7500

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

27-07-2017

| Document brevet cité au rapport de recherche | Date de publication | Membre(s) de la famille de brevet(s) | Date de publication |
|---|---|---|---|
| US 2012203089 A1 | 09-08-2012 | US 2012203089 A1<br>US 2014275868 A1<br>US 2016371451 A1 | 09-08-2012<br>18-09-2014<br>22-12-2016 |
| WO 2007052255 A2 | 10-05-2007 | AUCUN | |
| WO 2005025413 A2 | 24-03-2005 | AT 532454 T<br>AU 2004272062 A1<br>AU 2010241506 A1<br>CA 2538038 A1<br>CA 2852974 A1<br>CA 2896407 A1<br>CN 1905835 A<br>DK 1662987 T3<br>EP 1662987 A2<br>EP 2319403 A2<br>ES 2374563 T3<br>JP 4603547 B2<br>JP 5255594 B2<br>JP 5635041 B2<br>JP 6117733 B2<br>JP 6139616 B2<br>JP 2007504905 A<br>JP 2010221042 A<br>JP 2013046759 A<br>JP 2014221197 A<br>JP 2016027866 A<br>KR 20070020374 A<br>KR 20120041270 A<br>KR 20120101181 A<br>KR 20130006539 A<br>KR 20140002090 A<br>KR 20140134338 A<br>KR 20150117711 A<br>NZ 546432 A<br>NZ 580449 A<br>PT 1662987 E<br>US 2005100937 A1<br>US 2006062852 A1<br>US 2006182738 A1<br>US 2011166553 A1<br>US 2016058323 A1<br>WO 2005025413 A2 | 15-11-2011<br>24-03-2005<br>09-12-2010<br>24-03-2005<br>24-03-2005<br>24-03-2005<br>31-01-2007<br>27-02-2012<br>07-06-2006<br>11-05-2011<br>17-02-2012<br>22-12-2010<br>07-08-2013<br>03-12-2014<br>19-04-2017<br>31-05-2017<br>08-03-2007<br>07-10-2010<br>07-03-2013<br>27-11-2014<br>25-02-2016<br>21-02-2007<br>30-04-2012<br>12-09-2012<br>16-01-2013<br>07-01-2014<br>21-11-2014<br>20-10-2015<br>26-02-2010<br>30-06-2011<br>14-02-2012<br>12-05-2005<br>23-03-2006<br>17-08-2006<br>07-07-2011<br>03-03-2016<br>24-03-2005 |
| WO 2005102441 A1 | 03-11-2005 | AUCUN | |

EPO FORM P0460

Pour tout renseignement concernant cette annexe : voir Journal Officiel de l'Office européen des brevets, No.12/82

## RÉFÉRENCES CITÉES DANS LA DESCRIPTION

**Documents brevets cités dans la description**

- US 7972296 B2

**Littérature non-brevet citée dans la description**

- **JINGWEI SHAO.** *PLOS ONE,* vol. 7 (10), e48127